# EUROPEAN PATENT SPECIFICATION

(11) **EP 3 193 628 B1**
(45) Date of publication and mention of the grant of the patent: **01.04.2020**
(21) Application number: 15759531.5
(22) Date of filing: 14.08.2015
(51) Int. Cl.: A23K 40/20, A23K 10/10, A23K 10/33, A23K 10/30, A23K 20/174, A23K 20/158, A23K 10/14, A23K 20/20, A23K 50/10, A23K 40/25, A23K 50/15

(54) **PRODUCTION OF A PELLETED RUMINANT ANIMAL FEED**
HERSTELLUNG EINES PELLETIERTEN FUTTERS FÜR WIEDERKÄUER
PRODUCTION D'ALIMENTS GRANULÉS POUR ANIMAUX RUMINANTS

(30) Priority: 14.08.2014 GB 201414477
(43) Date of publication of application: 26.07.2017
(73) Proprietor: Harbro Limited, Turriff, Aberdeenshire AB53 4PA (GB)
(72) Inventor: THOMSON, William Anderson McColl, Turriff Aberdeenshire AB53 4PA (GB); HARLEY, Bryan Patrick, Turriff Aberdeenshire AB53 4PA (GB)
(74) Representative: Murgitroyd & Company
(86) International application number: PCT/GB2015/052372
(87) International publication number: WO 2016/024136

(56) References cited:
- GB-A- 1 520 577
- GB-A- 2 141 316
- US-A- 3 873 734
- DEHGHAN-BANADAKY ET AL: "Effects of barley grain processing on productivity of cattle", ANIMAL FEED SCIENCE AND TECHNOLOGY, ELSEVIER, AMSTERDAM, NL, vol. 137, no. 1-2, 1 September 2007 (2007-09-01), pages 1-24, XP022157526, ISSN: 0377-8401, DOI: 10.1016/J.ANIFEEDSCI.2006.11.021 & I B Mandell ET AL: "THE EFFECTS OF BARLEY PROCESSING ON NUTRIENT DIGESTION WITHIN THE GASTROINTESTINAL TRACT OF BEEF CATTLE FED MIXED DIETS", Canadian Journal of Animal Science, 1 March 1988 (1988-03-01), pages 191-98, XP55223031, Retrieved from the Internet: URL:http://pubs.aic.ca/doi/pdf/10.4141/cja s88-018 [retrieved on 2015-10-22]
- OWENS F N ET AL: "Acidosis in Cattle: A Review", JOURNAL OF ANIMAL SCIENCE, AMERICAN SOCIETY OF ANIMAL SCIENCE, US, vol. 76, 1 January 1998 (1998-01-01), pages 275-286, XP003014122, ISSN: 0021-8812

## Description

### Field of the invention

The present invention relates to a pellet composition of ruminant feed which reduces the risk of acidosis in a rumen, in turn reducing the time period at which the rumen may be below pH 5.5 and a process of making such ruminant feed.

### Background of the invention

Ruminants are unique in that they do not directly digest the feedstuffs they consume. Instead pre-gastric fermentation occurs whereby metabolic end products of digestion and low levels of consumed feedstuffs reach the small intestine. Ruminal microbes then act on carbohydrates to give volatile fatty acids and utilise non-protein nitrogen, for example, ammonia, urea, proteins, peptides and amino acids, to provide microbial or bacterial protein of high value. The microbial protein then passes out of the rumen and is subjected to gastric digestion in the abomasum of the ruminant and is absorbed in the small intestine.

The inherent characteristics or properties of feedstuff consumed by the ruminants such as moisture, pH, starch content and availability, good protein level and fat content can have a pronounced effect on ruminal pH, microbial protein production and ultimate growth potential of the animal.

It is known in the art that cereal grains require to be processed to maximise their availability for digestion prior to consumption by ruminants. This processing increases the efficiency with which the ruminants convert the grain into weight gain or increased milk production. However, it has been found that feeding such high digestive efficiency feed grains can lower ruminal pH values due to faster rates of volatile fatty acid production. While ruminal pH levels typically fluctuate between approximately 5.5 and 7 with normal ruminant diets, in diets containing significant proportions of high efficiency feed grains, ruminal pH levels may reach below pH 5.5 for periods of time. A pH between 5 and 5.5 is generally classed as sub-acute acidosis, whilst periods below pH 5 are classed as clinical acidosis. Animals may die when ruminal pH drops below 5 for extended periods.

Products such as Maxammon™ (Harbro) have been utilised to treat whole crop or grain at harvest as a whole crop or grain-only preservative and have been found to minimise damage to the rumen associated with acidosis. Treatment of grain with urea plus a urease enzyme premix to produce alkaline ammonia-treated grain has proven extremely successful. The treated grain can be used in blends or as a straight feed. In addition to acting as a whole crop and grain-only preservative, and minimising damage to the rumen normally associated with acidosis, alkaline ammonia-treated feed has also been indicated to provide an increase in the protein content (30% +) of the treated grain. Other known products such as Alkapellets® are manufactured alkaline compound feeds for ruminant feeding based on Alkagrain® 150 concentrate as the key raw material. Alkagrain ®150 concentrate is manufactured by releasing ammonia from Home n'Dry pellets into whole or processed cereal grains.

Typically pelleted compositions allow for reduced wastage of feedstuffs, improved palatability, less segregation in the feed, and provide for shorter eating periods. This in turn can lead to improved growth and uniformity in the animals being fed. Additionally, they can negate feeders from having to mix or provide nutritional admixtures and this is labour saving.

However, conventional pelleting processes focus on finely milling ingredients prior to extrusion into a pellet. This provides the optimal pellet durability to retain physical integrity of the pellet during storage, production, transport and subsequent use on-farm. However, this milling process (commonly through a 3mm screen) increases the surface area of ground cereal grains in the pellet leading to rapid fermentation in the animals' rumen. Traditionally, grinding within the compound feed process increases risk of both sub-acute and clinical acidosis particularly for high performance animals consuming large quantities of feed.

### Summary of the Invention

The present inventors have determined a process to utilise alkaline ammonia-treated grains and/or feed ingredients such that they can be incorporated into compound compositions or pellets. Recent trials with alkaline ammonia-treated grains or feed ingredients in compound compositions or pellets indicate that their use can significantly reduce risk from acidosis. Therefore the present invention relates to the use of alkaline ammonia-treated grains and/or feed ingredients in the production of a pelleted or compounded composition.

Whilst the use of alkaline ammonia-treated grain or feed ingredients, for use as a straight feed or for use in a blended grain feed has demonstrated advantages, it would be further advantageous to be able to utilise such alkaline ammonia-treated grain to provide pelleted or compounded compositions (pellets), to minimise the risk of sub-acute and clinical acidosis.

Accordingly, a first aspect of the present invention provides a process for the production of pelleted or compounded compositions (pellet) in accordance with claim 1.

Pellets or compound feeds provided by this process are considered to reduce the risk of acidosis in ruminants provided with the pelleted or compounded compositions. Without wishing to be bound by theory, it is considered that the alkaline ammonia-treatment process can for example allow hydrolysis of urea provided to the grain or feed ingredient to produce ammonia such that the ammonia reacts with the grain or feed ingredient, binding to the fibre, and allows slow release of ammonia in the rumen after intake of the grain or feed ingredient by the animal to be fed. This slow release of ammonia provides a source of buffering in the rumen and further provides a regulated release of nitrogen for bacterial growth. Further, this slow release of ammonia in the rumen, which increases the buffer capacity in the rumen, decreases the proportion of time the pH falls below 5.5, thus reducing acidosis risk.

The alkaline ammonia-treatment to provide alkaline treated grain or feedstuffs / feed ingredients is by treatment with urea and enzymes (for example urease either in purified form or as part of another component in the feed, in particular plant urease enzymes (although synthetic urease enzymes can also be used)) to hydrolyse the urea to ammonia, for example urea and plants containing urease. In embodiments a urease enzyme composition can be used at a rate of 5000mg/kg of grain or feedstuff to be treated wherein the enzyme composition has a typical urease activity of 5000µgN/g/min. These nitrogenous treatments allow the grain or feed ingredient to potentially release ammonia when they are combined with urease enzymes.

Typical high performance pelleted compound feeds contain levels of starch in excess of 15% from a range of cereal sources. In a normal circumstance, dry grain which would typically have an average moisture content of about 12% (12% to less than 14% moisture content would be the desirable average range) would be delivered to the mill, stored in a form of silo in this dry form and then incorporated into the feed via a grinding and mixing process. In the present invention, prior to grinding and mixing of the grain and / or feed ingredients, an alkaline (ammonia)- treatment process is performed to the grain and / or feed ingredients and the grain / feed ingredients are stored at 16% to 26%, moisture level, as for example measured using Near Infrared (NIR) spectroscopy or an Oven Method of dry matter assay, prior to the subsequent processing of the grain or feed ingredients to form the pellets. Such a process results in grain and / or feed ingredients with a pH greater than 8 with the alkaline effect arising from the action of ammonia on the grain / feed ingredient. The inventors have determined that alkaline ammonia treated grain and/or feed ingredient can be successfully processed to form pelleted or compounded compositions. Suitably pelleted or compounded compositions of the invention can have a pH of 6.0, suitably pH 6.5 or greater.

In embodiments alkaline ammonia-treated grain or feed ingredient can be provided by providing a urea and urease enzyme composition to the grain or feed ingredients and then storing the treated grain or feed ingredients, optionally following the addition of water to the grain or feed ingredients, for a period of greater than three days at a grain moisture content in the range 16% to 24%. As will be appreciated, the production of ammonia from the urea and urease enzyme composition will depend on the amounts of urea and urease provided, and the moisture content. Other factors may also influence the rate and amount of ammonia produced and thus, alkaline ammonia-treated grain or feed ingredients may be provided in less than three days, for example 2 days under suitable conditions.

In embodiments the step of providing alkaline ammonia-treated grain or feed ingredient can be the step of providing alkaline ammonia-treatment to grain and storage of the treated grain for at least three days prior to mixing of the treated grain and/or feed ingredient with other components of the pellet. Suitably, storage of the treated grain and/or feed ingredients may be at a moisture content in the range 16% to 24% for at least three days.

Suitably, following alkaline ammonia-treatment, the grain and/or feed ingredients may be stored in a pit, bin, or silo where the temperature and humidity of the grain may be monitored to minimise the risk of the grain spoiling e.g. germination or infestation with moulds, bacteria, mites etc. Treated grain is generally more stable as the raised pH has a preserving effect and deters the growth of most mould and bacteria species, and can deter vermin.

In embodiments the moisture level of the grains or feed ingredients to be treated or the alkaline ammonia-treated grains or feed ingredients may be adjusted to provide a moisture content in the range 16% to 24% after the urea and urease enzyme composition has been applied to the grain and or feed ingredients being treated. Suitably alkaline ammonia-treated grains (or feed ingredients) may be stored for at least three days, suitably for at least 7 days, suitably for 10 days, suitably between 1 to 2 weeks, prior to use in the grinding and mixing process to provide compound / pellet feed. Typically, storage can be provided under cover. Suitably treated grain is stable and may be stored for extended periods prior to use.

In embodiments alkaline ammonia-treatment can be by treatment with urea plus a natural source of urease enzyme or with any other nitrogenous source such that reaction of the urea and urease leads to the presence of ammonia in the grain or feed ingredient. In embodiments the alkaline ammonia-treatment can be provided to the grain and/or feed ingredients during the milling process, for example liquid ammonia can be provided to the grain and/or feed ingredient prior or during the mixing process or on or after forming the pellets.

In embodiments of the process, to provide alkaline ammonia-treated grain and/or feed ingredient, the urea plus a source of urease enzyme (for example urease present in or purified from suitable plants) is provided to grain at a concentration to allow the urea to be hydrolysed to ammonia in the desired time frame, as would be readily understood and/or determined based on the activity of the urease under the treatment conditions, to provide treated grain (or following addition of another nitrogenous source leading to the presence of ammonia in the grain or feed ingredient) and the treated grain and/or feed ingredient can be stored at a moisture content of 16% to 24% for a period of at least three days prior to being incorporated into a compound composition or pellet. This treatment period prior to use of the treated grain and/or feed ingredient in incorporation into compound or pellet feed is out with the period of a normal grain handling process.

Suitably, alkaline ammonia-treatment may be applied to whole grain or the grain may first be broken using conventional rolling or grinding technologies. In embodiments, at least 30% of the treated grain can retain a size of at least 5mm as determined by sieving.

Whilst any source of grain can be suitably treated, the invention preferably utilises a grain selected from barley, wheat, maize or oats or a combination thereof as a source for treatment. The resultant alkaline ammonia-treated grains are typically above the moisture content of grain conventionally used to produce compound/pellet feed.

Alternatively other feed materials or ingredients may be treated to provide the pelleted product with alkalinity benefit. For example, ammonia treatment will increase the energy value of high fibre feeds, for example fibrous byproducts of cereal and oil productions, for example oat husks, rice husks, rice bran, wheat fibre, wheat feed, sunflower and thus could be used as a method of uprating their energy and protein within the feed.

Typically, processing of grain increases the surface active area of grain available for degradation by rumen microbes and increases the speed of starch degradation. Increasing the speed of starch digestion may lead to increased levels of volatile fatty acids e.g. propionic acid, and a subsequent reduction in rumen pH.

Combining alkaline ammonia-treated grains or feed ingredients, suitably urea and urease treated grain, in a pellet subjected to non-conventional grinding (screen size greater or equal to 5 mm) results in a feed with unique properties. This feed can be particularly advantageous to buffer against pH reduction, directly raise pH and achieve a slower starch digestion than is experienced with a conventional pelleted feed. Pellets made according to the invention thus protect against acidosis and sustain improved production levels as a result of optimal rumen function.

Suitably, in embodiments, the present invention, provides for the use of coarsely ground (greater than 30% of particles at least or over 5mm) alkaline ammonia-treated grains and/or feed ingredients.

In such embodiments, the pellets may have a noticeably coarser structure typically with portions of grain being clearly visible in the compound feed.

Whilst it is known to add urea to dry grain as a preservative, or as a source of concentrated nitrogen, such urea is rapidly solubilised and often excreted before it can be captured by the rumen microbes. Thus, when urea is provided alone in the grain to the animal it is considered it will fail to raise rumen pH and the nitrogen may be inefficiently captured as microbial protein. By mixing the urea and a urease enzyme source together in a moist environment (suitably 16% to 24% moisture) and providing the treated grain and / or feed ingredient (for example a fibrous plant material) in a storage period, in embodiments for at least or greater than 3 days, preferably greater than 7 days, suitably 10 days, suitably between 1 to 2 weeks, prior to use of the grain and / or feed ingredient in the pelleting process, the urea is hydrolysed and produces large amounts of ammonia gas. The ammonia gas is solubilised in the moisture surrounding the grain/ feed ingredient and reacts with the carbohydrate to produce stable nitrogenous compounds. These provide a slow release of nitrogen within the rumen when the compound feed/ pellet produced using the treated grain and / or feed ingredient is fed to an animal and it has been determined that the grain in the pellet is thus able to modify rumen pH in the ruminant provided with the pellet. As noted above, the rate and level of ammonia produced can be influenced by environmental factors such as moisture content and thus an increase in the level of moisture can lead to an increase in the rate of ammonia production such that suitably alkaline ammonia-treated grains can be produced in less than 3 days, for example 1 or 2 days.

Alkaline ammonia-treated grains or feed ingredients demonstrate a rise in crude protein content commensurate with the level of urea added. Such protein content can be determined by any suitable means in the art, for example near infrared spectroscopy, or Kjeldahl analysis for nitrogen. Typically treated grains or feed ingredients will indicate a higher dry matter protein content than non-treated grains or feed ingredients.

In embodiments, the alkaline ammonia treated grain and / or feed ingredient can be stored in a sealed silo for about 10-14 days prior to use. This can provide an optimal period of time to allow a hydrolysis reaction to occur and the binding of ammonia to the grain / feed ingredient as it is solubilised by the moisture. Suitably, treated grain / feed ingredient may have a pH higher than 8, suitably higher than 8.5. Suitably the pH of the grain / feed ingredient prior to mixing and following alkaline treatment is around pH 8 - 9. Suitably the grain to be used in the mixing process can be coarsely processed prior to mixing or screened to determine a suitable particle size, for example equal to or greater than 6mm screen.

Suitably, alkaline ammonia-treated grain can be selected from for example, maize, barley, wheat, oats, rye, rice, triticale, and the like or combinations of such grain to be formed into pellets. Alternatively fibrous feed ingredients may be alkaline ammonia treated to provide fibrous raw material, for example oat husks, for inclusion in pellets. In embodiments, the alkaline ammonia-treated grain may comprise flaked maize. In embodiments, the alkaline ammonia-treated grain may be wheat or barley

In embodiments the alkaline ammonia-treated grain or feed ingredient can be provided by providing an alkaline or ammonia treatment to a grain when the grain has a moisture content in the range of 5%-28%, 10%-28%, 16%-28%, 14%-25%, 14%-16%, or around 18%. Rapid assay moisture sensors as are known in the art are readily available and widely used on farm and in grain stores. Alternatively, oven moisture measurement may be used to determine moisture content of grain or feed ingredients. Typically, mature grain is harvested when it includes a moisture level of preferably between 14-25%.

Optionally, during the process to provide the treated grain or feed ingredients, water can be added to the grain or feed ingredient being treated prior to, during or after the treatment. Suitably, water may be added to the grain to provide a moisture level in the range 16% to 24%, suitably 18% to 22%. A moisture content in this range allows ammonia, for example produced from hydrolysis of the urea, to bind to the grain or feed ingredient.

Typically mixing and pelleting steps can be carried out in known commercial equipment used in conventional pelleting processes. Mixing can be achieved, for example, using a revolving agitating apparatus. Suitably the ingredients are mixed for a time sufficient to allow a uniform mix of the components to be provided.

Suitably, as the moisture content of the alkaline ammonia-treated grain and/or feed ingredient is greater than observed in dry grain or feed ingredient, typically used to form pellets, less moisture, for example steam is required to be added during mixing and pelleting. Suitably moisture is provided to provide a moisture level during mixing or pelleting of about 11-13% moisture content.

In embodiments liquid ammonia can be added to the grain or feed ingredients during the production of the pellets or after the production of the pellets. The provision of liquid ammonia during or after production of the pellets can provide alkalinity within the feed. Without wishing to be bound by theory, provision of liquid ammonia would not be considered to provide the same level of reaction with structural carbohydrates leading to improved fibre digestibility and the slow release of ammonia into the rumen as would be provided by providing ammonia by providing urea and urease enzyme to the grain or feed ingredient prior to processing of the grain or feed ingredient to form a pellet.

Suitably, using the process of the present invention where alkaline ammonia-treatment has been provided to the grain or feed ingredients prior to mixing there is no requirement for additional ammonia or alkaline treatment to be provided to the finished pellets or to the pellets during their production, but this optional step may be included.

Following mixing of a pellet mixture the pellet mixture may be conditioned using conventional means, a pellet may be formed by compressing the mix to form pellets. In embodiments, pelleting can be performed by a ring dye pellet mill. Suitably a plurality of pellets may be formed from the pellet forming mixture. The soft pellets can then be dried and cooled. Pellets may be provided of a cylindrical shape and can be approximately 2.5 to 16 mm, 3 to 15 mm in diameter, suitably 4 to 14 mm in diameter. In embodiments the pellets can be of length 5 to 50 mm. Suitably, drying may be provided in a cooler or drier to provide pellets with a final moisture content about 11-16%, preferably below 14%, ideally 12%.

Durability of the finished pellets may be determined according to Pellet Durability Index (PDI) (Holmen) and Kahl tests.

Optionally, minerals, including for example copper, manganese, selenium, cobalt, zinc, mineral complexes, chelating or proteinated trace minerals, antibiotics, fibre sources, probiotics, amino acids and vitamins or combinations thereof may be added to the pellet forming mixture. Optionally the process can comprise a step of providing a buffer, a salt, sodium bicarbonate, magnesium carbonate, calcium, magnesium oxide, inorganic acid absorbing material, bentonite, Rumitech™ or combinations thereof to the pellet.

Disclosed herein is a pellet produced by the process of the first aspect of the invention.

Disclosed herein the pellet produced by the process of the first aspect of the invention can be in the range 2.5 to 16 mm, particularly 4 to 16mm diameter, 5 to 50 mm, in particular, 10 to 30mm long. In embodiments the durability of the pellet can be in the range Holmen 85+, Kahl 7 + to Holmen 96+, Kahl 12+.

Disclosed herein the pellet produced by the process can be a combination of cereals (0-100%), food co-products (0-80%), proteins (0-50%) to a total of 100%, more suitably cereals (10-100%), more suitably (10-80%), food co-products (20-80%), proteins (5-50%) with oils/fats, molasses and minerals, vitamins and trace elements. The alkaline (ammonia)-treated grain or feed ingredient, for example, urea and urease enzyme treated materials, can be added in a range from 5-80%, suitably 5-60%, for example 70%.

Disclosed herein a pellet of the present invention may be admixed with conventional pellets which are not formed following alkaline ammonia-treatment of the grain or feed ingredients.

Disclosed herein is a method to reduce the risk of rumen acidosis in a ruminant by decreasing the number of periods and / or length of time of such periods which the rumen pH falls below pH 5.5, the method comprising the step of providing an effective amount of pellet(s) produced by the process of the first aspect of the invention to the ruminant.

Suitably, clinical ruminal acidosis is characterised by acidification of the rumen, i.e. a low pH of the rumen liquor, usually below, but not limited to, pH 5 leading to, but not limited to, a reduction in fibre digestion and reduced dry matter intake. Sub-acute ruminal acidosis (SARA) and acute (clinical) acidosis can lead to disturbances in the composition of the ruminal flora and lead to damage of the rumen papillae and / or mucosa and may also lead to rapid decline in the health and performance of the animal and / or animal death.

The term ruminant in the invention relates to an animal from the subordination of cloven hoofed animals which are characterised by a multi-part ruminant stomach.

In embodiments ruminants can include cows, goats, sheep, camels, llamas, giraffes, bison, buffalos, deer, and antelope.

Preferred features and embodiments of each aspect of the invention are as for each of the other aspects mutatis mutandis unless context demands otherwise.

Throughout the specification, unless the context demands otherwise, the terms 'comprise' or 'include', or variations such as 'comprises' or 'comprising', 'includes' or 'including' will be understood to imply the includes of a stated integer or group of integers, but not the exclusion of any other integer or group of integers.

### Brief Description of the Figures

Examples of the present invention will now be exemplified by way of example only as illustrated in the following figures:

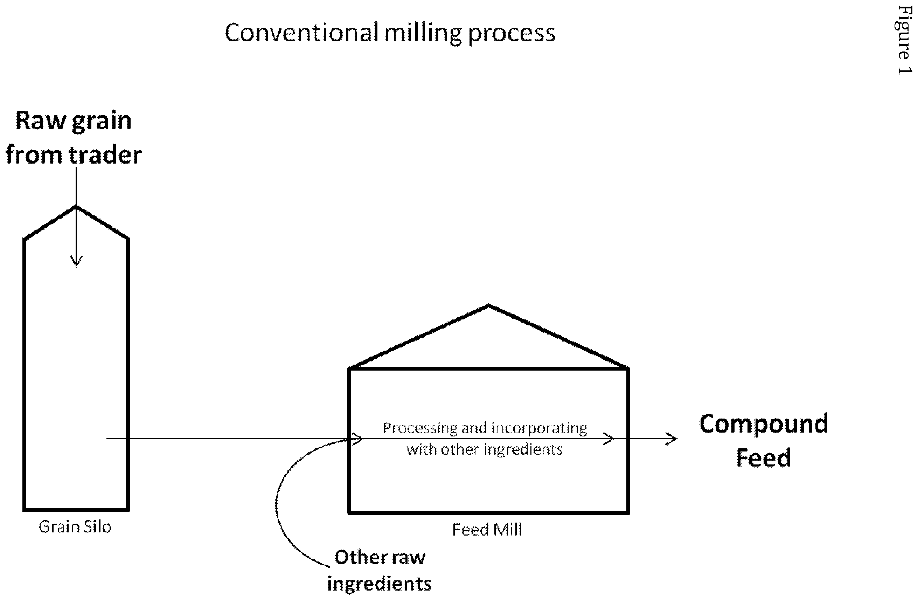

Figure 1 shows a conventional milling process.

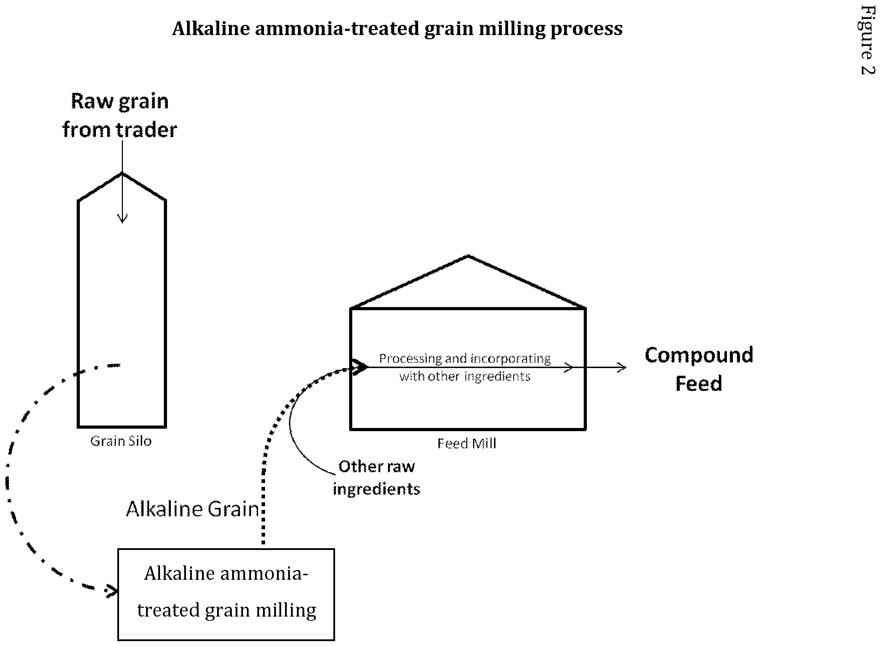

Figure 2 shows a milling process wherein the grain has been treated prior to milling such that urea and urease enzyme has been provided to the grain to provide alkaline grain.

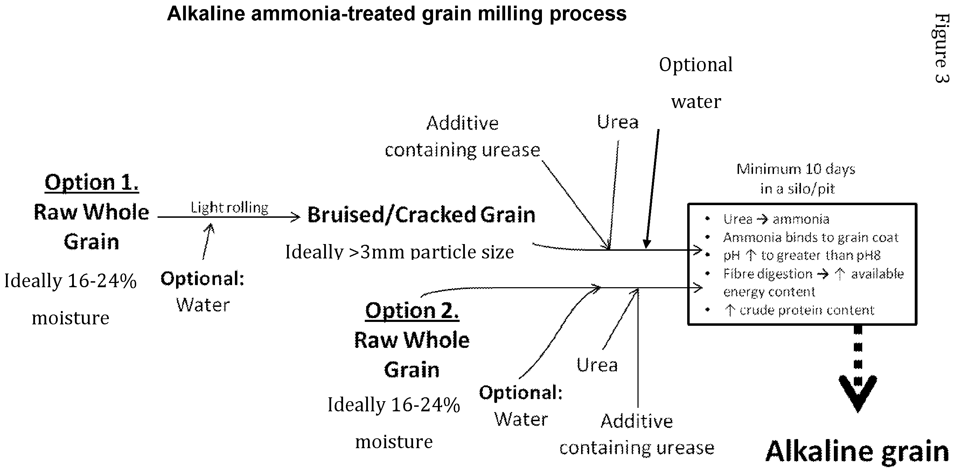

Figure 3 provides details of the treatment process prior to milling wherein urea is converted to ammonia, ammonia binds to the grain and the pH increases to greater than pH 8 leading to improved digestibility fibre digestion and an increase in the available energy content and increase in the crude protein content.

### Brief description of the Invention

A pellet of the present invention may be formed using a process according the following stages:
1. Weighing according to a formulation, a mixture of alkaline ammonia-treated feed ingredients along with a balanced quantity of proteins, carbohydrates, minerals, vitamins, liquids, antibiotics, probiotics, yeast cultures or amino acids;
2. Mixing the formulation and grinding when required, prior to warming under pressure as a conditioning process;
3. Compressing and extruding the mixture into a pellet.

Typically, alkaline ammonia-treatment is by treatment of grain and / or feed ingredients to be incorporated into the pellet with urea and urease and storage of the grain and / or feed ingredient for at least three days wherein the grain and / or feed ingredient have a moisture level of about 16% to 26%, most suitably about 18% such that ammonia produced by hydrolysis of the urea is able to bind to the grain and / or feed ingredient.

Typically mixing and pelleting apparatus used in the process are as known in the art and as used in conventional pelleting processes. Processing to form pellets includes subjecting the pellet forming mixture to steam prior to introduction of the pellet forming mixture or formulation into a pellet extruder. As is known in the art, the addition of steam can improve production rates, reduce dye wear and improve pellet quality. In the conditioning step, live steam may be injected into the pellet forming mixture as it is conveyed through the conditioner. The conditioner can comprise a cylindrical tube with a rotating shaft upon which paddles may be mounted. The conditioning steam can increase the temperature and moisture content of the pellet forming mixture. In embodiments, the conditioner can be pressurised allowing the use of higher temperatures and longer conditioning times to improve pellet durability and increase the production rate. The pressure and heat conditions for forming the pellet depend on the composition of the pellet and the desired characteristics of the pellet as would be understood in the art.

In examples of the process the addition of steam during the pelleting process may be adjusted to take account of the increased moisture content of the treated grain or feed ingredient. This adjustment can be made during the production process to ensure standardised production parameters.

### Examples

### Example 1

Typically to treat the grain, mature grain is harvested when it includes a moisture level of between 16-24%. Grain outside this moisture range (above and below) can be treated for example with urea and a urease enzyme composition, but the amount of treatment provided may differ from that provided below as would be known in the art, and optionally water may be added during storage of the grain to allow the hydrolysis reaction to proceed.

Application of the alkaline ammonia- treatment was provided as follows. In a mixer, 1000 kg of grain was provided to the mixer and 100kg of urea and urease enzyme composition and approximately 250kg weight of water were mixed together.

The grain and /or feed ingredient with the urea and urease composition was then mixed for between 1-2 minutes to activate the hydrolysis reaction. The remaining grain or feed ingredient (4000 kg) was then added and mixed for a further 3-4 minutes.

After mixing the resultant 5 tonnes of moist mixture (moisture content in the range 16 to 24%) was discharged and stored in a sealed silo for 1-2 weeks. Following the initial treatment period the grain may be exposed to air by opening the silo. At this time the treated product has a distinct smell of ammonia.

After treatment, the treated grain may be utilised in the mixing and pelleting process as described herein.

Where appropriate, the formulation or parts thereof may be ground through a hammer mill using specific parameters, for example screen, size, speed to create a mixture of a desired particle size spectrum.

The treated grain can then be provided to a mixer as part of a formulation as depicted in Table 1, together with the ranges within which the individual components can be varied.

**Table 1**

| **Ingredient** | **Normal Pellet %** | **Alkaline-ammonia treated Pellet %** |
|---|---|---|
| Whole Barley | 23 | 0 |
| Whole Maize | 3 | 3 |
| Wheatfeed | 22 | 21 |
| Maize Dark Grains | 22 | 21 |
| Oat Hulls | 2.5 | 2.5 |
| Extracted Rapeseed Meal | 11 | 9 |
| Rumen-Protected Rapeseed | 2.5 | 2.5 |
| Extracted High Protein Soya | 2.5 | 2.5 |
| Soya Hulls | 0 | 1.8 |
| Fat Spray | 0.2 | 0.2 |
| Rumen-Protected Fat | 0.2 | 0.2 |
| Sugar Cane Molasses | 8 | 8 |
| Limestone Flour | 2 | 2 |
| Calcined Magnesite | 0.5 | 0.5 |
| Salt | 0.5 | 0.5 |
| Trace Element & Vitamin Premix Alkaline-ammonia treated Whole | 0.5 | 0.5 |
| Wheat | 0 | 25 |

These would provide:

**Table 2**

| | **Normal Pellet % Analysis** | **Maxammon™ Pellet % Analysis** |
|---|---|---|
| [VOLUME] | | |
| Dry Matter | 86.50 | 85.77 |
| Oil, Ether Extract | 4.23 | 3.97 |
| Oil, AH | 5.24 | 5.01 |
| Crude Protein | 17.30 | 17.62 |
| Undegradable Dietary Protein | 5.27 | 5.32 |
| Fibre | 7.17 | 6.86 |
| Neutral Detergent Fibre (NDF) | 25.79 | 23.27 |
| Ash | 8.20 | 8.00 |
| Calcium | 1.25 | 1.16 |
| Phosphorous | 0.62 | 0.60 |
| Salt | 0.88 | 0.84 |
| Sodium | 0.35 | 0.34 |
| Chloride | 0.55 | 0.50 |
| Magnesium | 0.54 | 0.52 |
| Rumen Metabolisable Energy (ME) | 10.70 | 10.84 |
| Starch | 22.49 | 25.62 |
| Sugar | 8.00 | 7.94 |
| Vitamin A | 10.00 | 10.00 |
| Vitamin D3 | 2.00 | 2.00 |
| Vitamin E | 125.00 | 125.00 |
| Selenium | 0.42 | 0.40 |
| Copper | 32.54 | 31.13 |

All of the ingredients in the above formulation were mixed in a mixer for a period of time to get a uniform mix. For example the ingredients were weighed out according to the above formula and discharged from the weigher into a commercial feed mixer. The feed was then thoroughly mixed for several minutes to ensure even distribution of all ingredients.

After mixing the pellet forming mixture was delivered to the pellet mill feeder conditioning chamber wherein steam was added to the formulation and the formulation further mixed. The conditioned pellet forming mixture was then provided to a die extruder and the conditioned pellet forming mixture compacted through the die to form soft, moist pellets. The extruded pellets were then provided to a pellet cooler, which is designed to extract heat and surplus moisture from product to improve the physical quality of the pellet and allow its storage.

## Claims

1. A process for the production of pelleted compositions comprising the steps;
i) treating grain and / or feed ingredient with a urea and urease enzyme composition,
ii) storing the urease enzyme treated grain and/or feed ingredient at 16% to 26% moisture level such that the reaction of the urea and urease leads to a presence of ammonia in the grain or feed ingredient to provide alkaline ammonia-treated grain and/or feed ingredient with pH greater than 8,
iii) mixing and combining the alkaline ammonia-treated grain and/or feed ingredient of step ii) with the other components of the pellet to provide a pellet forming mixture,
iv) subjecting the pellet forming mixture to steam; and
providing at least one pellet from the pellet forming mixture through extrusion.

2. The process of claim 1 wherein the urea and urease enzyme composition and grain and/or feed ingredient is stored, optionally following the addition of water to the grain or feed ingredient, for a period of greater than three days at a moisture level in the range of 16% to 24%.

3. The process of any preceding claim wherein liquid ammonia is provided to the grain and/or feed ingredient prior to or during at least one of the mixing step, on and after forming the pellets.

4. The process of any preceding claim wherein at least 30% of the alkaline ammonia-treated grain and / or feed ingredient retains a size of at least 5mm as determined by sieving.

5. The process of any preceding claim wherein the alkaline ammonia-treated grain is selected from maize, barley, wheat, oats, rye, rice, triticale, or combinations of such grain to be formed into pellets.

6. The process of any preceding claim wherein grain is selected from barley, wheat, maize or oats or a combination thereof as a source for treatment.

7. The process of any preceding claim wherein the at least one pellet provided by the pellet forming mixture has a pH of 6.5 or greater.

## Patentansprüche

1. Ein Verfahren zur Herstellung von pelletierten Zusammensetzungen, beinhaltend die folgenden Schritte:
i) Behandeln von Getreide und/oder einem Futtermittelbestandteil mit einer Harnstoff- und Ureaseenzym-Zusammensetzung,
ii) Aufbewahren des mit Ureaseenzym behandelten Getreides und/oder Futtermittelbestandteils bei einem Feuchtigkeitsgrad von 16 % bis 26 %, sodass die Reaktion des Harnstoffs und der Urease zu einer Anwesenheit von Ammoniak in dem Getreide oder in dem Futtermittelbestandteil führt, um mit alkalischem Ammoniak behandeltes Getreide und/oder einen mit alkalischem Ammoniak behandelten Futtermittelbestandteil mit einem pH-Wert von mehr als 8 bereitzustellen,
iii) Mischen und Kombinieren des mit alkalischem Ammoniak behandelten Getreides und/oder mit alkalischem Ammoniak behandelten Futtermittelbestandteils von Schritt ii) mit den anderen Komponenten des Pellets, um eine pelletformende Mischung bereitzustellen,
iv) Aussetzen der pelletformenden Mischung gegenüber Dampf; und Bereitstellen von mindestens einem Pellet aus der pelletformenden Mischung durch Extrusion.

2. Verfahren gemäß Anspruch 1, wobei die Harnstoff- und Ureaseenzym-Zusammensetzung und das Getreide und/oder der Futtermittelbestandteil, optional nach Zugabe von Wasser zu dem Getreide oder dem Futtermittelbestandteil, über einen Zeitraum von mehr als drei Tagen bei einem Feuchtigkeitsgrad im Bereich von 16 % bis 24 % aufbewahrt wird.

3. Verfahren gemäß einem vorhergehenden Anspruch, wobei flüssiges Ammoniak vor oder während mindestens einem von dem Mischungsschritt, bei und nach dem Formen der Pellets dem Getreide und/oder Futtermittelbestandteil bereitgestellt wird.

4. Verfahren gemäß einem vorhergehenden Anspruch, wobei mindestens 30 % des mit alkalischem Ammoniak behandelten Getreides und/oder Futtermittelbestandteils eine Größe von mindestens 5 mm beibehält wie durch Sieben bestimmt.

5. Verfahren gemäß einem vorhergehenden Anspruch, wobei das mit alkalischem Ammoniak behandelte Getreide ausgewählt ist aus Mais, Gerste, Weizen, Hafer, Roggen, Reis, Tritikale oder Kombinationen aus solchem Getreide, um zu Pellets geformt zu werden.

6. Verfahren gemäß einem vorhergehenden Anspruch, wobei das Getreide ausgewählt ist aus Gerste, Weizen, Mais oder Hafer oder eine Kombination davon als eine Behandlungsquelle.

7. Verfahren gemäß einem vorhergehenden Anspruch, wobei das mindestens eine durch die pelletformende Mischung bereitgestellte Pellet einen pH-Wert von 6,5 oder mehr aufweist.

## Revendications

1. Un procédé pour la production de compositions granulées
comprenant les étapes :
i) traiter du grain et/ou de l'ingrédient d'aliment avec une composition d'urée et d'enzyme uréase,
ii) stocker le grain et/ou l'ingrédient d'aliment traités par enzyme uréase à un niveau d'humidité de 16 % à 26 % de manière à ce que la réaction de l'urée et de l'uréase conduise à une présence d'ammoniac dans le grain ou l'ingrédient d'aliment pour fournir du grain et/ou de l'ingrédient d'aliment traités par ammoniac alcalin avec un pH supérieur à 8,
iii) mélanger et combiner le grain et/ou l'ingrédient d'aliment traités par ammoniac alcalin de l'étape ii) avec les autres constituants du granulé pour fournir un mélange formant des granulés,
iv) soumettre le mélange formant des granulés à de la vapeur ; et
fournir au moins un granulé à partir du mélange formant des granulés par extrusion.

2. Le procédé de la revendication 1, dans lequel la composition d'urée et d'enzyme uréase et le grain et/ou l'ingrédient d'aliment est stocké, facultativement suivant l'ajout d'eau au grain ou à l'ingrédient d'aliment, pour une période supérieure à trois jours à un niveau d'humidité dans l'intervalle de 16 % à 24 %.

3. Le procédé de n'importe quelle revendication précédente, dans lequel de l'ammoniac liquide est fourni au grain et/ou à l'ingrédient d'aliment avant ou durant au moins une de l'étape du mélange, pendant et après la formation des granulés.

4. Le procédé de n'importe quelle revendication précédente, dans lequel au moins 30 % du grain et/ou de l'ingrédient d'aliment traités par ammoniac alcalin conserve une dimension d'au moins 5 mm telle que déterminée par tamisage.

5. Le procédé de n'importe quelle revendication précédente, dans lequel le grain traité par ammoniac alcalin est sélectionné parmi le maïs, l'orge, le blé, l'avoine, le seigle, le riz, le triticale, ou des combinaisons de tels grains pour être formé en granulés.

6. Le procédé de n'importe quelle revendication précédente, dans lequel du grain est sélectionné parmi l'orge, le blé, le maïs ou l'avoine ou une combinaison de ceux-ci en tant que source pour traitement.

7. Le procédé de n'importe quelle revendication précédente, dans lequel l'au moins un granulé fourni par le mélange formant des granulés a un pH de ou supérieur à 6,5.
